# FASCICULE DE BREVET EUROPEEN

(11) **EP 1 272 156 B1**
(45) Date de publication et mention de la délivrance du brevet: **31.05.2006**
(21) Numéro de dépôt: 01921509.4
(22) Date de dépôt: 06.04.2001
(51) Int. Cl.: A61K 8/34, A61K 8/40, A61Q 5/00, A61Q 19/00

(54) **UTILISATION D'INHIBITEURS DE L'ALCOOL DESHYDROGENASE DANS LE TRAITEMENT COSMETIQUE DES MATIERES KERATINIQUES**
VERWENDUNG VON ALKOHOLDEHYDROGENASE-INHIBITOREN IN DER KOSMETISCHEN BEHANDLUNG VON KERATINMATERIAL
USE OF ALCOHOL DEHYDROGENASE INHIBITORS FOR COSMETIC TREATMENT OF KERATINOUS MATERIALS

(30) Priorité: 12.04.2000 FR 0004709
(43) Date de publication de la demande: 08.01.2003
(73) Titulaire: L'OREAL, 75008 Paris (FR)
(72) Inventeur: SAINT-LEGER, Didier, F-92400 Courbevoie (FR)
(74) Mandataire: Casalonga, Axel
(86) Numéro de dépôt international: PCT/FR2001/001054
(87) Numéro de publication internationale: WO 2001/078677

(56) Documents cités:
- EP-A- 0 008 171
- EP-A- 0 412 705
- WO-A-96/23490
- DE-A- 4 229 707
- DE-A- 4 339 486
- DE-A- 19 806 890
- US-A- 5 252 322
- US-A- 5 304 379
- US-A- 5 616 150
- US-A- 5 665 367
- US-A- 5 686 100
- US-A- 5 705 145
- US-A- 5 853 742

## Description

La présente invention est relative à l'utilisation d'agents inhibiteurs de l'enzyme alcool déshydrogénase dans le traitement cosmétique des matières kératiniques, pour prévenir, atténuer et/ou supprimer les effets indésirables d'alcools primaires au niveau des couches superficielles de la peau et de ses annexes.

L'utilisation de ces agents inhibiteurs de l'enzyme alcool déshydrogénase permet également de lutter contre le vieillissement prématuré des tissus cutanés dû aux alcools primaires.

Les alcools primaires de structure générale R-CH₂OH comme l'éthanol, par exemple, sont des agents couramment utilisés dans les produits cosmétiques. Ils sont utilisés dans la plupart des cas comme véhicules à titre de solvants, mais ils peuvent aussi être utilisés pour leurs propriétés particulières comme le glycérol, par exemple, qui.est utilisé pour l'hydratation cutanée.

Il a été constaté que les alcools primaires altéraient les structures et fonctions des couches superficielles de la peau sans que ces modifications puissent être nécessairement détectables à l'oeil nu. A long terme, ces altérations entraînent des dessèchements ou des desquamations accélérées sur la surface cutanée.

On a constaté notamment des altérations de la microcirculation susceptibles d'entraîner des rougeurs et des érythèmes.

Il a été établi que ces affections cutanées sont dues aux aldéhydes formés suite à la catalyse enzymatique d'alcools primaires par l'enzyme alcool déshydrogénase. (Jonathan K. Wilkin, M.D. Journal of Investigation Dermatology, 1980, V.91, N°2, Pages 117-119).

Les effets de l'enzyme alcool déshydrogénase ont été également étudiés afin de traiter les ingestions d'éthylène glycol ou les toxicités dues à l'éthanol.

Les inhibiteurs de l'enzyme l'alcool déshydrogénase sont utilisés par ailleurs dans la recherche en biochimie ou dans un but thérapeutique, afin de lutter contre les empoisonnements à l'alcool (Biochemical and Biophysical research Communication, V.203, N°3, 1994, Septembre 30, 1994).

La demanderesse a découvert, que de plus, les alcools primaires agissent de manière négative sur les tissus de la couche superficielle de l'épidémie, prédisposant ainsi ces tissus à différents types d'agressions par les détergents terisio-actifs par exemple, et que l'utilisation d'inhibiteurs de l'enzyme alcool déshydrogénase dans des compositions cosmétiques contenant un alcool primaire permettait de prévenir, atténuer et/ou supprimer les effets indésirables d'alcools primaires au niveau des couches superficielles de la peau et de ses annexes.

L'invention a en particulier pour objet l'utilisation d'un inhibiteur de l'enzyme alcool déshydrogénase, afin de lutter contre le vieillissement prématuré des tissus cutanés dû aux effets nocifs des alcools primaires.

Un autre objet de la présente invention est un procédé de traitement cosmétique des matières kératiniques.

La présente invention porte également sur un agent de traitement cosmétique des matières kératiniques.

L'invention a également pour objet une composition cosmétique comprenant dans un milieu cosmétiquement acceptable contenant au moins un alcool primaire, au moins un inhibiteur de l'enzyme alcool déshydrogénase.

D'autres objets de l'invention apparaîtront à la lumière de la description et des exemples qui suivent.

L'invention porte sur l'utilisation d'un inhibiteur de l'enzyme alcool déshydrogénase, de préférence humaine, dans le traitement cosmétique des matières kératiniques, pour prévenir, atténuer et/ou supprimer les effets indésirables d'alcools primaires au niveau des couches superficielles de la peau et de ses annexes. On entend par couche superficielle de la peau et annexes, le stratum corneum, l'épiderme, le cuir chevelu, les follicules pileux, les glandes sébacées, les muqueuses, les glandes sudorales écrines ou apocrines.

Les inhibiteurs de l'enzyme alcool déshydrogénase peuvent notamment être utilisés afin de lutter contre le vieillissement prématuré des tissus cutanés dû aux effets indésirables des alcools primaires.

Les inhibiteurs de l'enzyme alcool déshydrogénase peuvent être présents dans une concentration de 0,0001 à 50% en poids, de préférence de 0,01 à 10% et encore plus préférentiellement de 0,1 à 10% par rapport au poids total de la composition.

Les inhibiteurs de l'enzyme alcool déshydrogénase choisis parmi l'auramine O, l'allicine, l'acide 1,5-anilino-naphtalène sulfonique, l'acide 1,7-anilino-naphtalène sulfonique, l'acide 1,8 anilino-naphtalène sulfonique, la berbérine, la canavanine, le 2,2'-diprypyl, l'imidazole, le m-méthylbenzamide, le 4-méthylpyrazole, le pyrazole, le 4-pentylpyrazole, l'O-phénantroline, l'alrestatine, l'acide anthranique, le O-carboxybenzaldéhyde, l'acide 2,3-diméthyl succinique, l'acide éthacrynique, l'acide isonicotinique, la phénacamide, la quercétine, la quercitrine, le sorbinil, l'acide tétraméthylène glutarique, l'acide valproïque, le propanolol, le 2,2,2-trichloroéthanol, le 4,5-diaminopyrazole et ses dérivés et le 2-éthyl-5-méthyl-2H-3,4-diaminopyrazole.

Selon un mode de réalisation préféré de l'invention, on utilise un inhibiteur conduisant à un pourcentage d'inhibition in vitro d'au moins 50% évalué au moyen du Kit Sigma Diagnostics (référence 333-B) avec une concentration d'éthanol de 5,75.10⁻⁵ M et une concentration de 10⁻³ M d'inhibiteur. Parmi les inhibiteurs préférés on peut citer le pyrazole, le 4-méthylpyrazole, l'imidazole, le 2,2,2-trichloroéthanol, le 4,5-diaminopyrazole et ses dérivés substitués sur les fonctions amines et/ou sur le cycle pyrazole et le 2-éthyl-5-méthyl-2H-3,4-diaminopyrazole.

Les inhibiteurs de l'enzyme alcool déshydrogénase définis précédemment peuvent être utilisés dans des compositions que l'on peut appliquer simultanément, conjointement ou de façon séquentielle avant ou après avec une composition cosmétique contenant au moins un alcool primaire.

De préférence, ils sont appliqués simultanément ou conjointement.

Les alcools primaires ont une structure générale R-CH₂OH dans laquelle R est une chaîne hydrocarbonée linéaire ou ramifiée, saturée ou insaturée, cyclique ou non cyclique, pouvant comporter un ou plusieurs hétéroatomes et un ou plusieurs autres groupes hydroxyles. Les hétéroatomes sont choisis de préférence parmi les atomes de soufre, d'oxygène ou d'azote. R désigne de préférence un radical alkyle linéaire ou ramifié ayant 1 à 30 atomes de carbone et de préférence 1 à 15 atomes de carbone.

Les alcools pouvant être utilisés selon l'invention sont l'éthanol, le n-propanol, le n-butanol, les polyéthylène glycols, le 1,3-propanediol, le dodécanol, l'alcool cétylique, l'alcool oléylique, l'alcool laurylique, l'alcool stéarylique, l'alcool myristylique, l'alcool béhénylique, l'alcool linoléylique, le glycérol et le 1,2-propanediol, ces deux derniers composés contenant à la fois des fonctions alcools primaire et secondaire.

Les compositions cosmétiques selon l'invention peuvent se trouver sous différentes formes, par exemple sous forme de lotions, de laits, de crèmes, de shampooings, de sprays, de mousses, de sticks, etc...

Les compositions cosmétiques de l'invention peuvent comprendre en outre, selon les applications auxquelles elles sont destinées, des adjuvants différents des alcools primaires définis ci-dessus choisis parmi les corps gras, les solvants organiques, les gélifiants, les émollients, les adoucissants, les antioxydants, les opacifiants, les stabilisants, les silicones, les agents anti-mousse, les agents - anti-hydratants, les vitamines, les parfums, les conservateurs, les agents tensio-actifs anioniques, cationiques, non-ioniques, amphotères, zwitterioniques ou leurs mélanges, les charges, les séquestrants, les polymères, les propulseurs, les agents alcanisants ou acidifiants, les colorants, les pigments, les agents épaississants, les anti-radicalaires, les filtres solaires ou tout autre adjuvant habituellement utilisé en cosmétique.

La composition cosmétique peut également être utilisée comme agent cosmétique sous forme de "kit". Ces agents cosmétiques comprennent au moins deux composants :
- un premier composant comprenant au moins un alcool primaire et
- un deuxième composant comprenant au moins un inhibiteur de l'enzyme alcool déshydrogénase,
ces deux composants étant destinés à être appliqués simultanément, conjointement ou de façon séquentielle sur les tissus cutanés destinés à être protégés.

La présente invention se référe également à un procédé de traitement cosmétique des matières kératiniques consistant en l'application d'au moins deux composants :
- un premier composant comprenant au moins un alcool primaire et
- un deuxième composant comprenant au moins un inhibiteur de l'enzyme alcool déshydrogénase,
ces deux composants étant destinés à être appliqués simultanément, conjointement ou de façon séquentielle sur les tissus cutanés destinés à être protégés.

La présente invention se réfère également à une composition cosmétique comprenant, dans un milieu cosmétiquement acceptable, au moins un alcool primaire tel que défini ci-dessus et au moins un inhibiteur de l'enzyme alcool déshydrogénase conduisant in vitro à un pourcentage d'au moins 50% d'inhibition de l'activité de l'alcool déshydrogénase.

Les compositions peuvent contenir des adjuvants cosmétiques tels que ceux mentionnés ci-dessus.

Les exemples qui suivent sont destinés à illustrer l'invention, sans pour autant présenter un caractère limitatif.

### EXEMPLES DE FORMULATIONS

**Lotion anti-chute des cheveux**

| | | |
|---|---|---|
| Ethanol | | 30 % |
| 2,2,2-trichloréthanol | | 5 % |
| 2,4-Diaminopyrimidine N-oxyde-3 | | 1,5 % |
| Parfum | | 0,5 % |
| Eau | qsp | 100 |

**Gel solaire à l'alcool**

| | | |
|---|---|---|
| Ethanol | | 8 % |
| Imidazole | | 3 % |
| Carbopol 980 | | 0,3 % |
| Triéthanolamine | | 0,4 % |
| Parahydroxybenzoate de méthyle | | 0,2 % |
| Eau | qsp | 100 |

**Shampooing à l'alcool**

| | | |
|---|---|---|
| Lauryl éther sulfate de sodium | | 16 % |
| Tégobétaïne | | 8 % |
| Ethanol | | 5 % |
| 4-méthylpyrazole | | 1 % |
| Dioléate de polyéthylèneglycol | | 3 % |
| Acide citrique | | 2,3 %: |
| Chlorure de sodium | | 0,9 % |
| Esters de l'acide para hydroxybenzoïque | | 0,5 % |
| Eau | qsp | 100 |

**Crème Hydratante**

| | | |
|---|---|---|
| Alcool cétylstéarylique et polyéthylène glycol éther d'alcool cétylstéarylique (dénomination CTFA: cetearyl alcohol et ceteareth 30) vendu sous la dénomination Nonidac 1618F par la société Condéa | | 7 % |
| Mono-distéarate de glycérol vendu sous la dénomination Géléol pastilles par la société Gattefosse | | 2 % |
| Alcool cétylique vendu sous la dénomination Lanette 16 NF par la société Cognis | | 1,5 % |
| Microémulsion de silicone vendue sous la dénomination DC200Fluid 350 cs par la société Dow Corning | | 1,5 % |
| Huile de vaseline vendue sous la dénomination Marcol 82 par la société Esso | | 15 % |
| P-hydroxybenzoate de butyle | | 0,2 % |
| Glycérine pure codex synthétique ou végétale | | 20 % |
| Pyrazole | | 4 % |
| Imidazolidinyl urée | | 0,2 % |
| Eau distillée | qsp | 100 |

### Test d'inhibition de l'activité de l'enzyme alcool déshydrogénase

L'inhibition de l'activité de l'enzyme alcool déshydrogénase a été déterminée au moyen du kit Sigma Diagnostics (Réf. 333-B).

3 ml du Réactif Alcool, 10 µl de solution d'éthanol (éthanol 0,08% p/v ref. 330-20) et 10 µl de solution d'inhibiteur dans l'eau distillée sont mis à incuber pendant 10 minutes à température ambiante.

Pour chaque dosage, un blanc est réalisé consistant en 3 ml du Réactif Alcool, 10 µl d'eau distillée et 10 µl de solution d'inhibiteur dans l'eau distillée.

Les inhibiteurs sont dilués de manière à se retrouver dans le mélange final à la concentration de 10⁻³ M.

Après incubation du mélange, 100 µl sont prélevés et lus dans un intervalle de 10 minutes à 340 nm. Les lectures sont réalisées en micro-plaques 96 puits UV au moyen d'un spectrophotomètre UV-visible multipuits (Spectramax Plus- Molecular Devices) contre le blanc.

Les résultats d'inhibition sont exprimés en pourcentage d'absorbance à 340 nm par rapport à une référence (absence d'inhibiteur dans le mélange) :

| **Inhibiteur** | % |
|---|---|
| Référence | 100 |
| Pyrazole | 16 |
| 4-méthylpyrazole | 35 |
| Imidazole | 32 |
| 2,2,2-trichloroéthanol | -12 |
| 2-éthyl-5-méthyl-2H-3,4-diaminopyrazole | 31 |

## Revendications

1. Utilisation d'un inhibiteur de l'enzyme alcool déshydrogénase dans le traitement cosmétique des matières kératiniques, pour prévenir, atténuer et/ou supprimer les effets indésirables d'alcools primaires au niveau des couches superficielles de la peau et de ses annexes.

2. Utilisation selon la revendication 1, **caractérisée par le fait que** les couches superficielles de la peau et annexes sont le stratum corneum, l'épiderme, le cuir chevelu, les follicules pileux, les glandes sébacées, les muqueuses, les glandes sudorales écrines ou apocrines.

3. Utilisation selon la revendication 1 ou 2, dans le but de lutter contre le vieillissement prématuré des tissus cutanés dû aux effets indésirables des alcools primaires.

4. Utilisation selon l'une quelconque des revendications 1 à 3, **caractérisée par le fait que** le composé inhibiteur de l'enzyme alcool déshydrogénase est présent dans une concentration de 0,0001 à 50 % en poids par rapport au poids total de la composition.

5. Utilisation selon l'une quelconque des revendications 1 à 4, **caractérisée par le fait que** le composé inhibiteur de l'enzyme alcool déshydrogénase est présent dans une concentration de 0,01 à 10 % en poids, et de préférence de 0,1% à 10% en poids, par rapport au poids total de la composition.

6. Utilisation selon l'une quelconque des revendications 1 à 5, **caractérisée par le fait que** le composé inhibiteur de l'enzyme alcool déshydrogénase est choisi parmi l'auramine O, l'allicine, l'acide 1,5-anilino-naphtalène sulfonique, l'acide 1,7-anilino-naphtalène sulfonique, l'acide 1,8-anilino-naphtalène sulfonique, la berbérine, la canavanine, le 2,2'-diprypyl, l'imidazole, le m-méthylbenzamide, le pyrazole, le 4-méthylpyrazole, le 4-pentylpyrazole, l'O-phénantroline, l'alrestatine, l'acide anthranique, le O-carboxybenzaldéhyde, l'acide 2,3-diméthyl succinique, l'acide éthacrynique, l'acide isonicotinique, la phénacamide, la quercétine, la quercitrine, le sorbinil, l'acide tétraméthylène glutarique, l'acide valproïque, le propanolol, le 2,2,2-trichloroéthanol, le 4,5-diaminopyrazole et ses dérivés et le 2-éthyl-5-méthyl-2H-3,4-diaminopyrazole.

7. Utilisation selon l'une quelconque des revendications 1 à 6, **caractérisée par le fait que** l'inhibiteur de l'enzyme alcool déshydrogénase conduit à un pourcentage d'inhibition in vitro d'au moins 50%.

8. Utilisation selon la revendication 7 d'un inhibiteur choisi parmi le pyrazole, le 4-méthylpyrazole, l'imidazole, le 2,2,2-trichloroéthanol le 4,5-diaminopyrazole et ses dérivés substitués sur les fonctions amines et/ou sur le cycle pyrazole et le 2-éthyl-5-méthyl-2H-3,4-diaminopyrazole.

9. Utilisation selon l'une quelconque des revendications 1 à 8, **caractérisée par le fait que** les inhibiteurs de l'enzyme alcool déshydrogénase sont appliqués sur les matières kératiniques simultanément, conjointement ou de façon séquentielle avec une composition cosmétique contenant au moins un alcool primaire.

10. Utilisation selon la revendication 9, **caractérisée par le fait que** l'alcool primaire répond à la formule R-CH₂OH dans laquelle R est une chaîne hydrocarbonée linéaire ou ramifiée, saturée ou insaturée, cyclique ou non cyclique, pouvant comporter un ou plusieurs hétéroatomes et un ou plusieurs autres groupes hydroxyles.

11. Utilisation selon la revendication 10, **caractérisée par le fait que** le radical R désigne un radical alkyle linéaire ou ramifié ayant 1 à 30 atomes de carbone.

12. Utilisation selon l'une quelconque des revendications 10 ou 11, **caractérisée par le fait que** les hétéroatomes sont choisis parmi les atomes de soufre, d'oxygène ou d'azote.

13. Utilisation selon l'une quelconque des revendications 9 à 12, **caractérisée par le fait que** l'alcool primaire est choisi parmi l'éthanol, le le n-propanol, le n-butanol, les polyéthylène glycols, le 1,3-propanediol, le dodécanol, l'alcool cétylique, l'alcool oléyque, l'alcool laurylique, l'alcool stéarylique, l'alcool mystirylique, l'alcool béhénylique, l'alcool linoléylique, le glycérol et le 1,2-propanediol.

14. Procédé de traitement cosmétique des matières kératiniques **caractérisé par le fait qu'**il consiste en l'application d'au moins deux composants:
- un premier composant comprenant au moins un alcool primaire, et
- un second composant comprenant au moins un inhibiteur de l'alcool déshydrogénase choisi dans le groupe constitué de 4-méthylpyrazole, le pyrazole, le 2,2,2-trichloroéthanol; et le 2-éthyl-5-méthyl-2H-3,4-diaminopyrazole,
ces deux composants étant appliqués simultanément, conjointement ou de façon séquentielle sur les tissus cutanés destinés à être protégés.

15. Agent cosmétique sous forme de kit comportant au moins deux composants:
- un premier composant comprenant au moins un alcool primaire, et
- un second composant comprenant au moins un inhibiteur de l'alcool déshydrogénase choisi dans le groupe constitué de 4-méthylpyrazole, le pyrazole, le 2,2,2- trichloroéthanol et le 2-ethyl-5-méthyl-2H-3,4-diaminopyrazole
ces deux composants étant destinés à être appliqués simultanément, conjointement ou de façon séquentielle sur les tissus cutanés destinés à être protégés.

16. Composition cosmétique comprenant dans un milieu cosmétiquement acceptable au moins un alcool primaire et au moins au moins un inhibiteur choisi dans le groupe constitué de 4-méthylpyrazole, le pyrazole, le 2,2,2-trichloroéthanol, et le 2-éthyl-5-méthyl-2H-3,4-diaminopyrazole,
conduisant in vitro à un pourcentage d'au moins 50% d'inhibition de l'activité de l'alcool déshydrogénase.

17. Composition cosmétique comprenant dans un milieu cosmétiquement acceptable au moins un alcool primaire choisi parmi le n-propanol, le n-butanol, les polyéthylène glycols, le 1,3-propanediol, le dodécanol, l'alcool oleylique, l'alcool laurylique, l'alcool myristylique, l'alcool bénéhylique, l'alcool linoléylique et au moins un inhibiteur
conduisant in vitro à un pourcentage d'au moins 50% d'inhibition de l'activité de l'alcool déshydrogénase.

18. Composition selon l'une quelconque des revendications 16 à 17, **caractérisée par le fait qu'**elle contient en outre au moins un adjuvant différent des alcools primaires définis ci-dessus choisis parmi les corps gras, les solvants organiques, les gélifiants, les émollients, les adoucissants, les antioxydants, les opacifiants, les stabilisants, les silicones, les agents anti-mousse, les agents anti-hydratants, les vitamines, les parfums, les conservateurs, les agents tensio-actifs anioniques, cationiques, non-ioniques, amphotères, zwitterioniques ou leurs mélanges, les charges, les séquestrants, les polymères, les propulseurs, les agents alcanisants ou acidifiants, les colorants, les pigments, les agents épaississants, les anti-radicalaires, les filtres solaires.

## Claims

1. Use of an inhibitor of the enzyme alcohol dehydrogenase in the cosmetic treatment of keratin materials, to prevent, alleviate and/or suppress the adverse effects of primary alcohols on the superficial layers of the skin and of its appendices.

2. Use according to Claim 1, **characterized in that** the superficial layers of the skin and of its appendices are the stratum corneum, the epidermis, the scalp, the hair follicles, the sebaceous glands, the mucous membranes and the eccrine or apocrine sweat glands.

3. Use according to Claim 1 or 2, with the aim of combating the premature ageing of skin tissues caused by the adverse effects of primary alcohols.

4. Use according to any one of Claims 1 to 3, **characterized in that** the compound that is an inhibitor of the enzyme alcohol dehydrogenase is present in a concentration from 0.0001 to 50% by weight, relative to the total weight of the composition.

5. Use according to any one of Claims 1 to 4, **characterized in that** the compound that is an inhibitor of the enzyme alcohol dehydrogenase is present in a concentration from 0.01 to 10% by weight and preferably from 0.1% to 10% by weight, relative to the total weight of the composition.

6. Use according to any one of Claims 1 to 5, **characterized in that** the compound which is an inhibitor of the enzyme alcohol dehydrogenase is chosen from auramine O, allicin, 1,5-anilinonaphthalenesulfonic acid, 1,7-anilinonaphthalenesulfonic acid, 1,8-anilinonaphthalenesulfonic acid, berberine, canavanine, 2,2'-diprypyl, imidazole, m-methylbenzamide, pyrazole, 4-methylpyrazole, 4-pentylpyrazole, O-phenanthroline, alrestatin, anthranic acid, O-carboxybenzaldehyde, 2,3-dimethylsuccinic acid, ethacrynic acid, isonicotinic acid, phenacamide, quercetin, quercitrin, sorbinil, tetramethyleneglutaric acid, valproic acid, propranolol, 2,2,2-trichloroethanol, 4,5-diaminopyrazole and its derivatives and 2-ethyl-5-methyl-2H-3,4-diaminopyrazole.

7. Use according to any one of Claims 1 to 6, **characterized in that** the compound that is an inhibitor of the enzyme alcohol dehydrogenase results in a percentage of inhibition in vitro of at least 50%.

8. Use according to Claim 7 of an inhibitor chosen from pyrazole, 4-methylpyrazole, imidazole, 2,2,2-trichloroethanol, 4,5-diaminopyrazole and its derivatives substituted on the amine functions and/or on the pyrazole ring, and 2-ethyl-5-methyl-2H-3,4-diaminopyrazole.

9. Use according to any one of Claims 1 to 8, **characterized in that** the inhibitors of the enzyme alcohol dehydrogenase are applied on the keratin materials simultaneously, concomitantly or sequentially with a cosmetic composition containing at least one primary alcohol.

10. Use according to Claim 9, **characterized in that** the primary alcohol corresponds to the formula R-CH₂OH, in which R is a linear or branched, saturated or unsaturated, cyclic or noncyclic, hydrocarbon-based chain, which may include one or more hetero atoms and one or more other hydroxyl groups.

11. Use according to Claim 10, **characterized in that** the radical R denotes a linear or branched alkyl radical containing 1 to 30 carbon atoms.

12. Use according to either of Claims 10 or 11, **characterized in that** the hetero atoms are chosen from sulfur, oxygen and nitrogen atoms.

13. Use according to any one of Claims 9 to 12, **characterized in that** the primary alcohol is chosen from ethanol, n-propanol, n-butanol, polyethylene glycols, 1,3-propanediol, dodecanol, cetyl alcohol, oleyl alcohol, lauryl alcohol, stearyl alcohol, myristyl alcohol, behenyl alcohol, linoleyl alcohol, glycerol and 1,2-propanediol.

14. Process for the cosmetic treatment of keratin materials, **characterized in that** it consists of the application of at least two components:
- a first component comprising at least one primary alcohol and
- a second component comprising at least one inhibitor of alcohol dehydrogenase chosen from the group made up of 4-methylpyrazole, pyrazole, 2,2,2-trichloroethanol and 2-ethyl-5-methyl-2H-3,4-diaminopyrazole,
these two components being applied simultaneously, concomitantly or sequentially on the skin tissues to be protected.

15. Cosmetic agent in the form of a kit, comprising at least two components:
- a first component comprising at least one primary alcohol and
- a second component comprising at least one inhibitor of alcohol dehydrogenase chosen from the group made up of 4-methylpyrazole, pyrazole, 2,2,2-trichloroethanol and 2-ethyl-5-methyl-2H-3,4-diaminopyrazole,
these two components being intended to be applied simultaneously, concomitantly or sequentially on the skin tissues to be protected.

16. Cosmetic composition comprising, in a cosmetically acceptable medium, at least one primary alcohol, and at least one inhibitor chosen from the group made up of 4-methylpyrazole, pyrazole, 2,2,2-trichloroethanol and 2-ethyl-5-methyl-2H-3,4-diaminopyrazole, resulting, in vitro, in an at least 50% inhibition of the activity of alcohol dehydrogenase.

17. Cosmetic composition comprising, in a cosmetically acceptable medium, at least one primary alcohol chosen from n-propanol, n-butanol, polyethylene glycols, 1,3-propanediol, dodecanol, oleic alcohol, lauryl alcohol, myristyl alcohol, behenyl alcohol, linoleyl alcohol and at least one inhibitor,
resulting, in vitro, in an at least 50% inhibition of the activity of alcohol dehydrogenasel.

18. Composition according to either of Claims 16 or 17, **characterized in that** it also contains at least one additive other than the primary alcohols defined above, chosen from fatty substances, organic solvents, gelling agents, emollients, softeners, antioxidants, opacifiers, stabilizers, silicones, antifoams, anti-hydrating agents, vitamins, fragrances, preservatives, anionic, cationic, nonionic, amphoteric or zwitterionic surfactants, or mixtures thereof, fillers, sequestering agents, polymers, propellants, basifying or acidifying agents, colorants, pigments, thickeners, free-radicals scavengers and sunscreens.

## Patentansprüche

1. Verwendung eines Inhibitors des Enzyms Alkohol-Dehydrogenase für die kosmetische Behandlung von Keratinsubstanzen, um den unerwünschten Wirkungen von primären Alkoholen auf die Oberflächenschichten der Haut und ihren Anhangsgebilden vorzubeugen, sie abzuschwächen und/oder sie zu unterdrücken.

2. Verwendung nach Anspruch 1, **dadurch gekennzeichnet, dass** es sich bei den Oberflächenschichten der Haut und der Anhangsgebilde um das Stratum corneum, die Epidermis, die Kopfhaut, die Haarfollikel, die Talgdrüsen, die Schleimhäute oder die ekrinen oder apokrinen Schweißdrüsen handelt.

3. Verwendung nach Anspruch 1 oder 2, um vorzeitige Hautalterung der Hautgewebe zu bekämpfen, die von den unerwünschten Wirkungen primärer Alkohole herrühren.

4. Verwendung nach einem der Ansprüche 1 bis 3, **dadurch gekennzeichnet, dass** der Alkohol-Dehydrogenase-Inhibitor in einer Konzentration von 0,0001 bis 50 Gew.-%, bezogen auf das Gesamtgewicht der Zusammensetzung, enthalten ist.

5. Verwendung nach einem der Ansprüche 1 bis 4, **dadurch gekennzeichnet, dass** der Alkohol-Dehydrogenase-Inhibitor in einer Konzentration von 0,01 bis 10 Gew.-% und vorzugsweise 0,1 bis 10 Gew.-%, bezogen auf das Gesamtgewicht der Zusammensetzung, enthalten ist.

6. Verwendung nach einem der Ansprüche 1 bis 5, **dadurch gekennzeichnet, dass** der Alkohol-Dehydrogenase-Inhibitor ausgewählt ist unter: Auramin O, Allicin, 1,5-Anilino-naphthalinsulfonsäure, 1,7-Anilino-naphthalinsulfonsäure, 1,8-Anilino-naphthalinsulfonsäure, Berberin, Canavanin, 2,2'-Diprypyl, Imidazol, *m*-Methylbenzamid, Pyrazol, 4-Methylpyrazol, 4-Pentylpyrazol, O-Phenantrolin, Alrestatin, Anthranilsäure, O-Carboxybenzaldehyd, 2,3-Dimethylbernsteinsäure, Ethacrynsäure, Isonicotinsäure, Phenacamid, Quercetin, Quercitrin, Sorbinil, Tetramethylenglutarsäure, Valproinsäure, Propanolol, 2,2,2-Trichlorethanol, 4,5-Diaminopyrazol und seinen Derivaten und 2-Ethyl-5-methyl-2H-3,4-diaminopyrazol.

7. Verwendung nach einem der Ansprüche 1 bis 6, **dadurch gekennzeichnet, dass** der Alkohol-Dehydrogenase-Inhibitor in vitro zu einer prozentualen Inhibierung von mindestens 50 % führt.

8. Verwendung nach Anspruch 7 eines Inhibitors, der unter Pyrazol, 4-Methylpyrazol, Imidazol, 2,2,2-Trichlorethanol, 4,5-Diaminopyrazol und seinen an den Aminofunktionen und/oder am Pyrazolring substituierten Derivaten und 2-Ethyl-5-methyl-2H-3,4-diaminopyrazol ausgewählt ist.

9. Verwendung nach einem der Ansprüche 1 bis 8, **dadurch gekennzeichnet, dass** die Alkohol-Dehydrogenase-Inhibitoren auf die Keratinsubstanzen gleichzeitig oder gemeinsam mit einer kosmetischen Zusammensetzung, die mindestens einen primären Alkohol enthält, oder getrennt von ihr aufgebracht werden.

10. Verwendung nach Anspruch 9, **dadurch gekennzeichnet, dass** der primäre Alkohol der Formel R-CH₂OH entspricht, worin R eine geradkettige oder verzweigte, gesättigte oder ungesättigte, cyclische oder nicht cyclische Kohlenwasserstoffkette ist, die ein oder mehrere Heteroatome und eine oder mehrere weitere Hydroxygruppen enthalten kann.

11. Verwendung nach Anspruch 10, **dadurch gekennzeichnet, dass** die Gruppe R eine geradkettige oder verzweigte Alkylgruppe mit 1 bis 30 Kohlenstoffatomen ist.

12. Verwendung nach einem der Ansprüche 10 oder 11, **dadurch gekennzeichnet, dass** die Heteroatome unter Schwefel, Sauerstoff oder Stickstoff ausgewählt sind.

13. Verwendung nach einem der Ansprüche 9 bis 12, **dadurch gekennzeichnet, dass** der primäre Alkohol unter *n*-Propanol, *n-*Butanol, Polyethylenglycolen, 1,3-Propandiol, Dodecanol, Cetylalkohol, Oleylalkohol, Laurylalkohol, Stearylalkohol, Myristylalkohol, Behenylalkohol, Linoleylalkohol, Glycerin und 1,2-Propandiol ausgewählt ist.

14. Verfahren zur kosmetischen Behandlung von Keratinsubstanzen, **dadurch gekennzeichnet, dass** es darin besteht, dass mindestens zwei Komponenten aufgetragen werden:
- eine erste Komponente, die mindestens einen primären Alkohol enthält, und
- eine zweite Komponente, die mindestens einen Alkohol-Dehydrogenase-Inhibitor enthält, der unter 4-Methylpyrazol, Pyrazol, 2,2,2-Trichlorethanol und 2-Ethyl-5-methyl-2H-3,4-diaminopyrazol ausgewählt ist,
wobei die beiden Komponenten gleichzeitig, gemeinsam oder nacheinander auf die zu schützenden Hautgewebe aufgetragen werden.

15. Kosmetisches Mittel in Kit-Form, das mindestens zwei Komponenten enthält:
- eine erste Komponente, die mindestens einen primären Alkohol enthält, und
- eine zweite Komponente, die mindestens einen Alkohol-Dehydrogenase-Inhibitor enthält, der unter 4-Methylpyrazol, Pyrazol, 2,2,2-Trichlorethanol und 2-Ethyl-5-methyl-2H-3,4-diaminopyrazol ausgewählt ist,
wobei die beiden Komponenten gleichzeitig, gemeinsam oder nacheinander auf die zu schützenden Hautgewebe aufgetragen werden.

16. Kosmetische Zusammensetzung, die in einem kosmetisch akzeptablen Medium mindestens einen primären Alkohol und mindestens einen Inhibitor enthält, der unter 4-Methylpyrazol, Pyrazol, 2,2,2-Trichlorethanol und 2-Ethyl-5-methyl-2H-3,4-diaminopyrazol ausgewählt ist und in vitro zu einer prozentualen Inhibierung der Aktivität der Alkohol-Dehydrogenase von mindestens 50 % führt.

17. Kosmetische Zusammensetzung, die in einem kosmetisch akzeptablen Medium mindestens einen primären Alkohol, der unter *n*-Propanol, *n*-Butanol, Polyethylenglycolen, 1,3-Propandiol, Dodecanol, Oleylalkohol, Laurylalkohol, Myristylalkohol, Behenylalkohol und Linoleylalkohol ausgewählt ist, und mindestens einen Inhibitor enthält, der in vitro zu einer prozentualen Inhibierung der Aktivität der Alkohol-Dehydrogenase von mindestens 50 % führt.

18. Zusammensetzung nach einem der Ansprüche 16 bis 17, **dadurch gekennzeichnet, dass** sie ferner mindestens einen Zusatzstoff enthält, der von den oben definierten primären Alkoholen verschieden ist und unter den Fettsubstanzen, organischen Lösungsmitteln, Gelbildnern, Emollientien, beruhigenden Stoffen, Antioxidantien, Trübungsmitteln, Stabilisatoren, Siliconen, Schaumverhütungsmitteln, Antihydratisierungsmitteln, Vitaminen, Parfums, Konservierungsmitteln, anionischen, kationischen, nichtionischen, amphoteren, zwitterionischen grenzflächenaktiven Stoffen oder deren Gemischen, Füllstoffen, Maskierungsmitteln, Polymeren, Treibmitteln, Alkalisierungsmitteln, Ansäuerungsmitteln, Farbmitteln, Pigmenten, Verdickungsmitteln, Radikalfängern für freie Radikale und Sonnenschutzfiltern ausgewählt ist.
